Europäisches Patentamt

European Patent Office

Office européen des brevets

(19)

(11) Publication number: **0 119 621**
**A1**

# EUROPEAN PATENT APPLICATION

(12)

(21) Application number: 84103000.0

(22) Date of filing: 19.03.84

(51) Int. Cl.³: **C 12 N 15/00,** C 12 P 19/34,
C 12 P 21/00
// C12R1/19

(30) Priority: 21.03.83 US 477210

(43) Date of publication of application: 26.09.84
Bulletin 84/39

(84) Designated Contracting States: AT BE CH DE FR GB IT
LI LU NL SE

(71) Applicant: F. HOFFMANN-LA ROCHE & CO.
Aktiengesellschaft, CH-4002 Basel (CH)

(72) Inventor: Bhatt, Ram S., 11 River Road, Nutley, N.J. (US)
Inventor: Crowl, Robert M., 246 Paterson Avenue, Little
Falls, N.J. (US)
Inventor: Poonian, Mohindar S., 16 Knoll Place, West
Caldwell, N.J. (US)

(74) Representative: Lederer, Franz, Dr. et al, Patentanwälte
Dr. Lederer Franz Meyer-Roxlau Reiner F.
Lucile-Grahn-Strasse 22, D-8000 München 80 (DE)

(54) Interleukin-2.

(57) A method for the preparation of mature human IL-2 by
direct expression of a synthetic gene coding therefor in a
transformed microorganism, corresponding DNA se-
quences, expression vehicles and microorganisms
therefor,

```
                                                              50
AATTCTATG GCTCCGACTT CAAGTTCTAC CAAGAAGACC CAGCTTCAAT
       GATAC CGAGGCTGAA GTTCAAGATG GTTCTTCTGG GTCGAAGTTA

                                                             100
TAGAACATTT ACTTCTAGAT TTACAAATGA TTCTGAATGG TATCAACAAT
ATCTTGTAAA TGAAGATCTA AATGTTTACT AAGACTTACC ATAGTTGTTA

                                                             150
TATAAGAATC CAAAGCTTAC TCGTATGTTG ACCTTTAAAT TCTATATGCC
ATATTCTTAG GTTTCGAATG AGCATACAAC TGGAAATTTA AGATATACGG

                                                             200
TAAGAAGGCT ACTGAATTAA AACACCTGCA GTGTTTAGAA GAAGAGCTCA
ATTCTTCCGA TGACTTAATT TTGTGGACGT CACAAATCTT CTTCTCGAGT

                                                             250
AACCGTTAGA AGAAGTTCTG AATCTGGCTC AATCTAAAAA CTTCCATTTA
TTGGCAATCT TCTTCAAGAC TTAGACCGAG TTAGATTTTT GAAGGTAAAT

                                                             300
CGTCCACGAG ATCTTATCTC TAATATTAAC GTAATCGTTT TGGAACTTAA
GCAGGTGCTC TAGAATAGAG ATTATAATTG CATTAGCAAA ACCTTGAATT

                                                             350
AGGATCCGAA ACTACCTTCA TGTGTGAATA TGCTGACGAA ACCGCTACGA
TCCTAGGCTT TGATGGAAGT ACACACTTAT ACGACTGCTT TGGCGATGCT

                                                             400
TCGTAGAATT TCTTAATCGA TGGATTACTT TCTGTGAATC TATTATCTCT
AGCATCTTAA AGAATTAGCT ACCTAATGAA AGACAGTTAG ATAATAGAGA

ACCTTAACTT GAGTCGACG
TGGAATTGAA CTCAGCTGCT TAA
```

---

ACTORUM AG

F. Hoffmann-La Roche & Co. Aktiengesellschaft
Basel / Schweiz

0119621

RAN 4105/77

Interleukin-2

Human interleukin-2 (IL-2) is a soluble protein which is capable of modulating lymphocyte reactivity and promoting the long-term in vitro culture of antigen-specific effector T-lymphocytes (mitogenesis) and, in the past, has been produced by stimulating mouse, rat or human lymphocyte cells with a mitogen.

IL-2 purified from mouse, rat and human normal T-lymphocytes has been found to exhibit different types of biological activities and may be useful in cases of T-cell depletion or to stimulate the formation of cytotoxic T-cells in some forms of cancer (Journal of The American Medical Association, Vol. 249, pages 166-171 [January 14, 1983]).

Human IL-2 derived from induced human malignant cells has recently been purified to homogeneity using multiple high performance liquid chromatography (HPLC) steps (S. Stern at the Third International Symposium of HPLC of Proteins, Peptides and Polynucleotides, November 14-16, 1983, Monte Carlo, Monaco. Title: Reverse-phase supports for the separation of polypeptides: Purification to homogeneity of IL-2 from a human T-cell leukemia.). The purified human IL-2 exhibits potent activity promoting the long-term in vitro culture of antigen-specific effector T-lymphocytes and modulating lymphocyte reactivity. A final purification of 500,000-fold was obtained with a specific activity of about $10^9$ U/mg and the following amino acid composition:

Mez/22.2.84

| Asx | 12.4 | Ala | 5.9 | Tyr | 3.0 |
|-----|------|-----|-----|-----|------|
| Thr | 11.6 | Cys | 3.3 | Phe | 5.8 |
| Ser | 7.4 | Val | 4.0 | His | 3.8 |
| Glx | 16.5 | Met | 4.0 | Lys | 10.6 |
| Pro | 7.4 | Ile | 8.5 | Arg | 4.9 |
| Gly | 2.4 | Leu | 20.8 | Trp | N.D. |

Dr. Taniguchi of the Cancer Research Institute, Tokyo, Japan, reported on the cloning and sequencing of the IL-2 gene and expression of the immature form of human interleukin-2 at the third Annual Recombinant DNA Congress in Philadelphia, Pennsylvania, on February 9, 1983. The cDNA from which the amino acid sequence of the IL-2 protein was derived was obtained from mRNA obtained from Jurkat cells induced with Con A. The full length cDNA clone is 800 base pairs long and codes for a protein of 153 amino acids. While Dr. Taniguchi provided a proposed amino acid sequence for the mature IL-2 protein and the sequence for the preceding signal·peptide, no suggestion was made for a method for converting the natural gene into a gene capable of expressing the mature form of the protein directly. Additionally, it should be noted that no convenient restriction sites were noted on the DNA sequence near the amino terminus portion. On March 24, 1983 Taniguchi et al. published in Nature 302, 305-310 on the structure and expression of a cloned cDNA for human IL-2.

### Summary of the Invention

The present invention deals with human interleukin-2 and a method of preparing it. More specifically the present invention provides

- a structural gene coding for mature human IL-2,
- a double stranded polydeoxyribonucleotide coding for mature human IL-2 having cohesive ends, each one representing a part of an endonuclease recognition site,

- 3 -

0119621

- replicable microbial expression vehicles capable, in a transformant microorganism, of expressing mature human IL-2,
- microorganisms transformed with these expression vehicles,
- mature human IL-2 with methionine as the first amino acid of the sequence (Met-IL-2) and
- a process for preparing structural genes and expression vehicles coding for mature human IL-2 and effecting the direct expression thereof in transformed microorganisms respectively.

The method of preparing structural genes coding for mature human IL-2 comprises

a) preparing a first series of oligodeoxyribonucleotide fragments which, when joined in the proper sequence, yield a DNA coding strand for the amino acid sequence of the mature human IL-2;

b) preparing a second series of oligodeoxyribonucleotide fragments which, when joined in the proper sequence, yield a DNA strand complementary to the coding strand;

c) effecting hydrogen bonding between mutually complementary portions of the first and second series of fragments to form a double-stranded structure and

d) completing the respective strands by ligation.

Finally, the present invention involves the isolation and purification from the fermentation broth of the desired recombinant mature human IL-2 so as to provide human IL-2 essentially free of bacterial protein and thus suitable for use as a therapeutic agent.

## Brief Description of the Drawings

The accompanying drawings illustrate aspects of preferred embodiments of the invention.

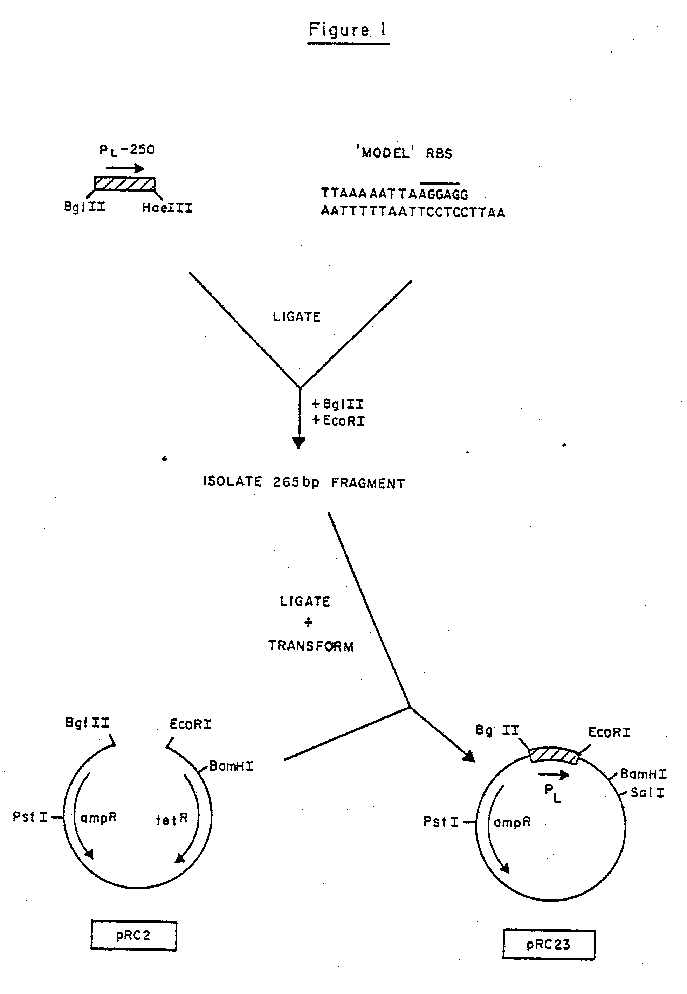

Figure 1. A schematic outline is described of the process by which a preferred cloning and expression vector promoting gene expression is derived from the bacteriophage lambda $P_L$ promoter. This vector, identified as pRC23, is constructed by ligating synthetic oligonucleotides containing a "consensus" (computer generated) ribosome binding site (Scherer et al., Nucleic Acids Research $\underline{8}$, 3895 [1980]) to a 250 bp Bgl II-Hae III fragment containing the $P_L$ promoter and inserting the ligation product into the plasmid pRC2. For further details concerning the construction of expression vector pRC 23 see European published patent application 99084.

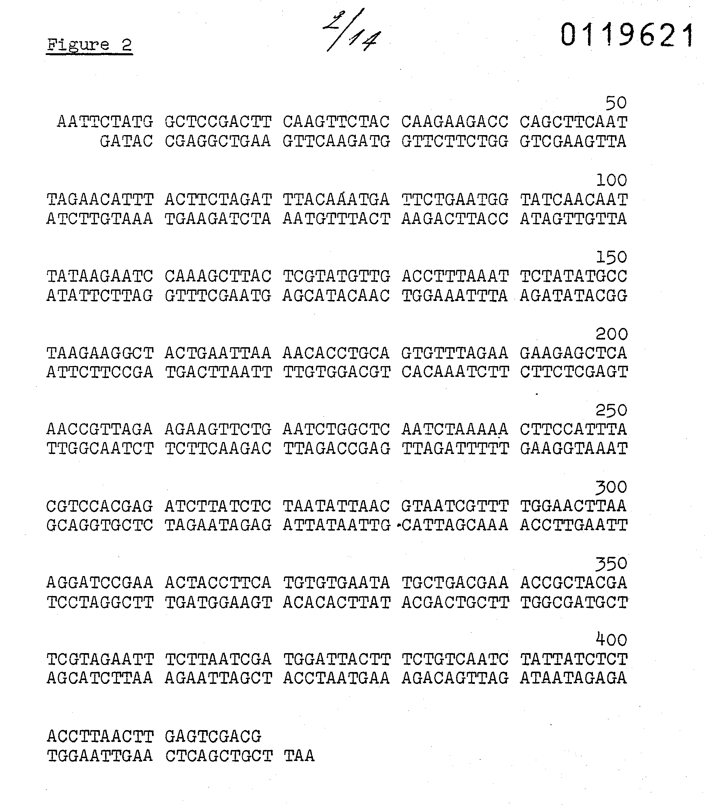

Figure 2 shows the double-stranded gene with restriction endonuclease termini at either end coding for mature human IL-2. This gene is suitable for insertion directly into the expression vector.

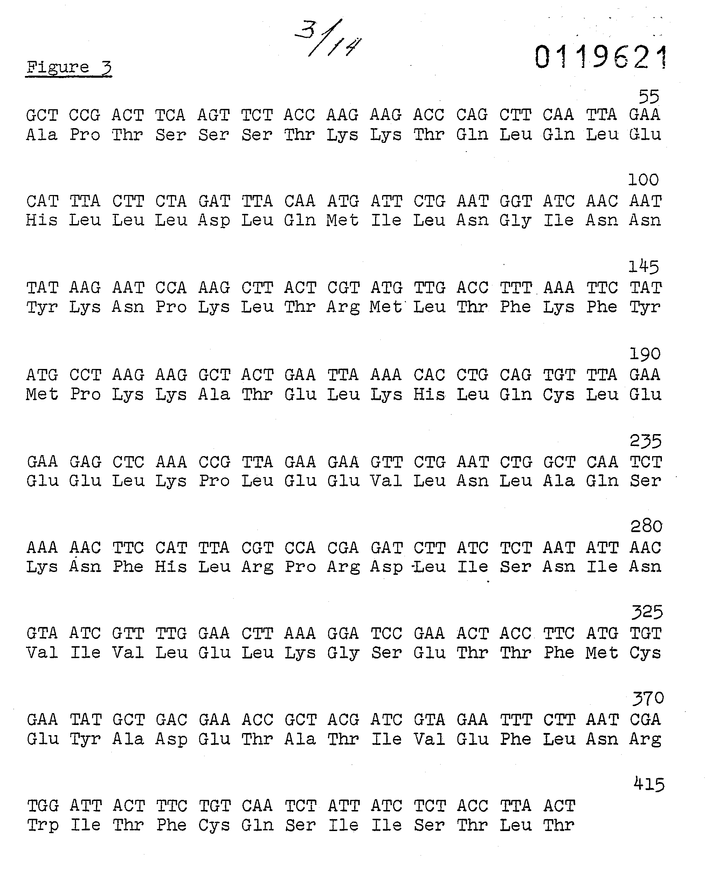

Figure 3. The amino acid sequence of mature human IL-2 is shown along with a DNA-sequence coding therefore.

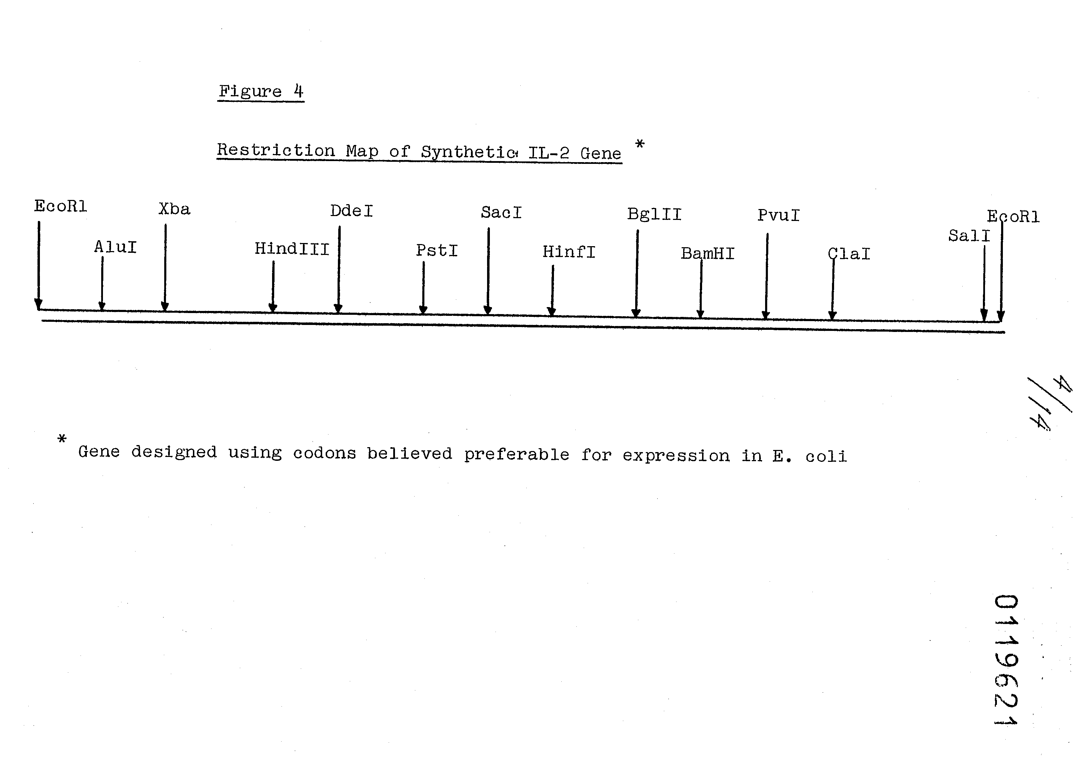

Figure 4 shows a restriction map of the synthetic structural gene for IL-2. It is seen that by proper selection of codons forming the synthetic gene a substantial number of restriction sites are available throughout the structural gene which is distinguishable from the natural gene.

Figure 5. A series of 40 oligodeoxyribonucleotide fragments which are employed in the construction of the synthetic gene for mature human IL-2 with restriction site termini is shown. The fragments with the odd numbers represent the coding strand the fragments with the even numbers the complementary gene strand.

Figure 6 shows the construction of the amino terminus portion of the synthetic gene for mature human IL-2. This portion is obtained by step-wise ligation of fragments 1-24.

Figure 7 shows the construction of the carboxy terminus portion of the synthetic gene for mature human IL-2. This portion is constructed utilizing fragments 25-40.

Figure 8. The ligation of the amino and carboxy terminal fragments of the synthetic gene for mature human IL-2 is schematically shown. A 6 base overhang is employed to effectuate the ligation.

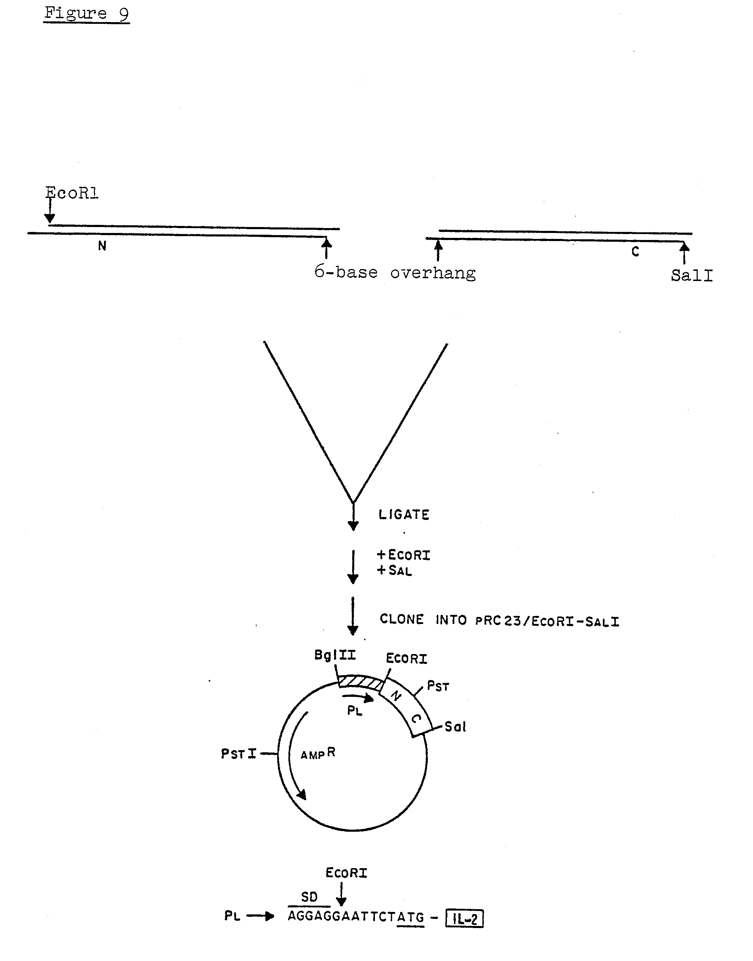

Figure 9. A flow chart for the construction of a recombinant plasmid capable of expressing mature human IL-2 beginning with the parenteral plasmid pRC 23 is shown. The gene for human IL-2 obtained in Figure 8 is inserted into the plasmid. $Amp^r$ denotes the gene for ampicillin resistance. The relative positions of various endonuclease restriction sites are shown. The gene for tetracycline resistance ($Tet^r$) normally found in pRC 23 is eliminated when the restriction cut at the SalI site is made. Thus the recombinant plasmid exhibits only ampicillin resistance for screening.

Figure 10. A schematic outline of the loading of the glass support utilized in the solid support phosphite methodology employed in the synthesis of oligonucleotides is described. The procedure is adapted from Matteucci and Caruthers, J.Am. Chem. Soc. <u>103</u>, 3185 (1981).

Figure 11. A schematic outline of the oligonucleotide synthesis using the solid support phosphite method is shown.

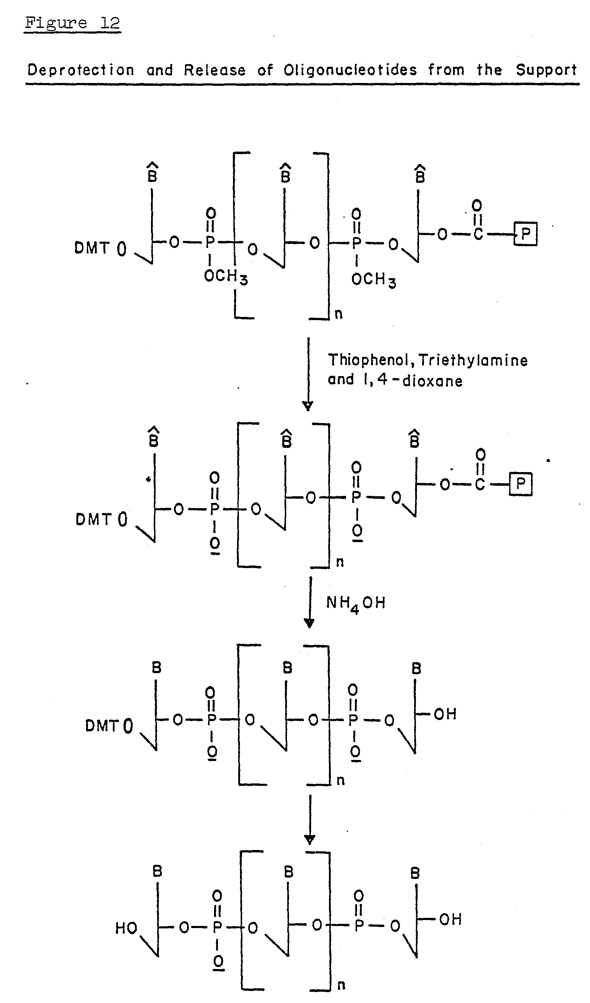

Figure 12. A schematic outline of deprotection and release of oligonucleotides from the solid support in the solid support phosphite method is depicted.

Figure 13. A generic representation of the functional intermediates used in the solid support triester methodology for the preparation of oligonucleotide fragments is shown. The procedure is adapted from Dembek et al., J. Am. Chem. Soc. <u>103</u>, 706 (1981).

Figure 14. A schematic outline of the reaction scheme employed in the construction of oligonucleotide fragments by the solid support triester method is shown.

<u>Detailed Description of the Invention</u>
<u>Preparation of Genes Coding for Mature Human IL-2</u>

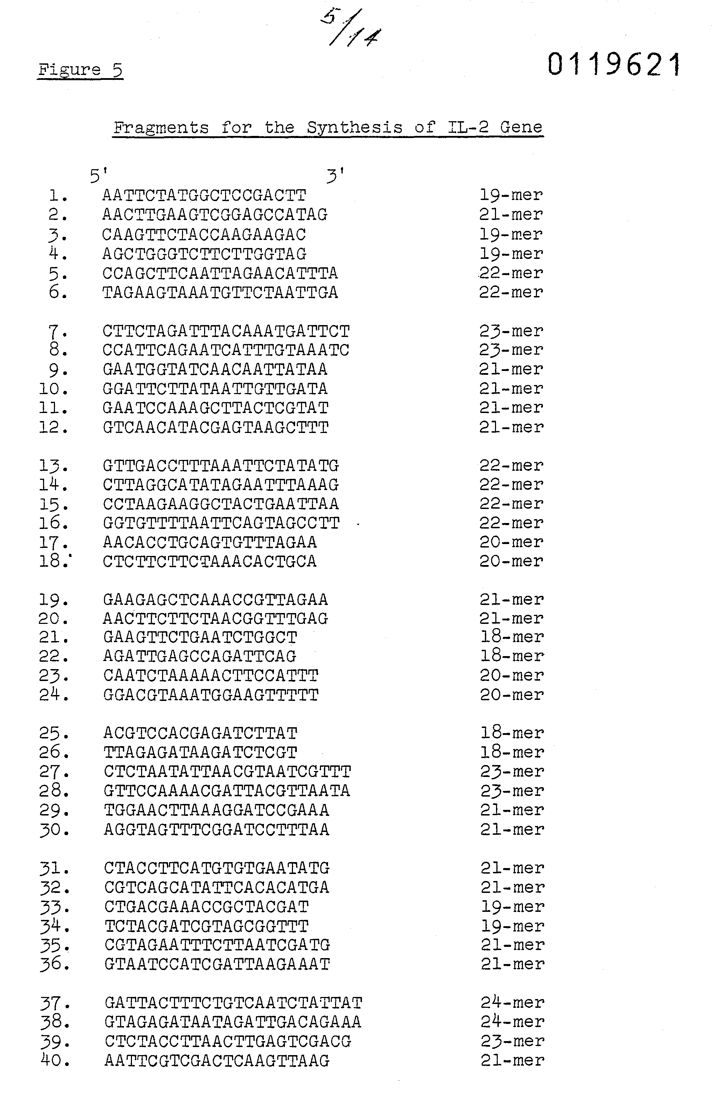

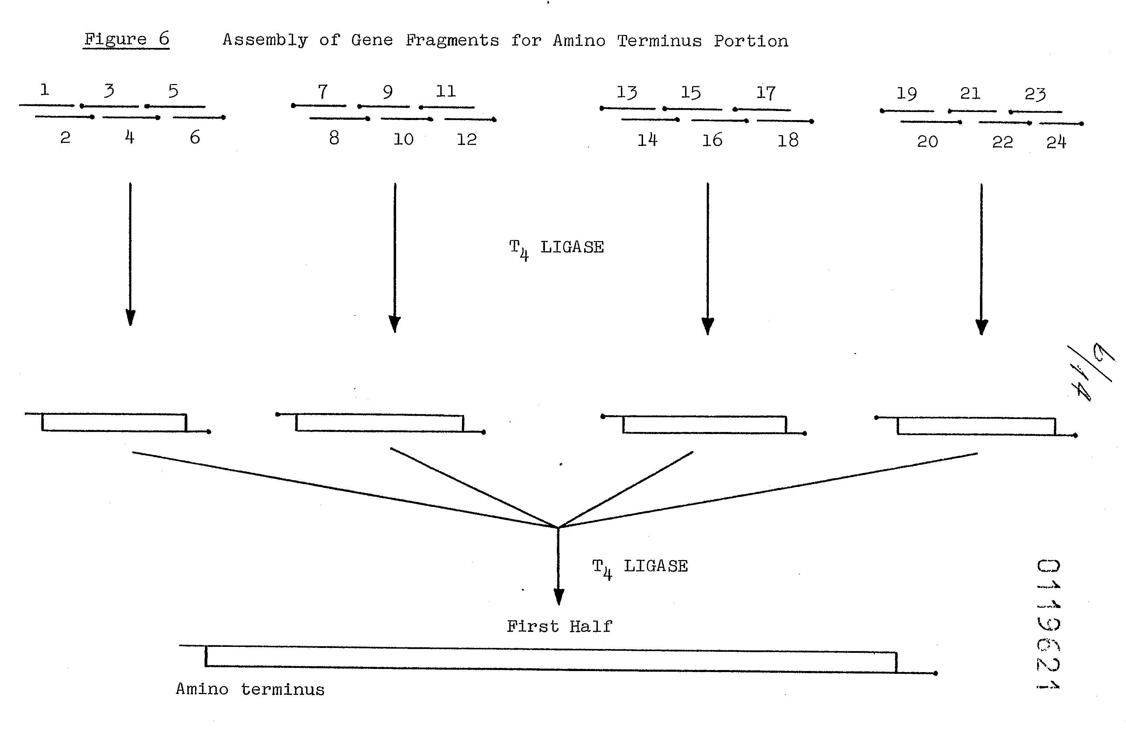

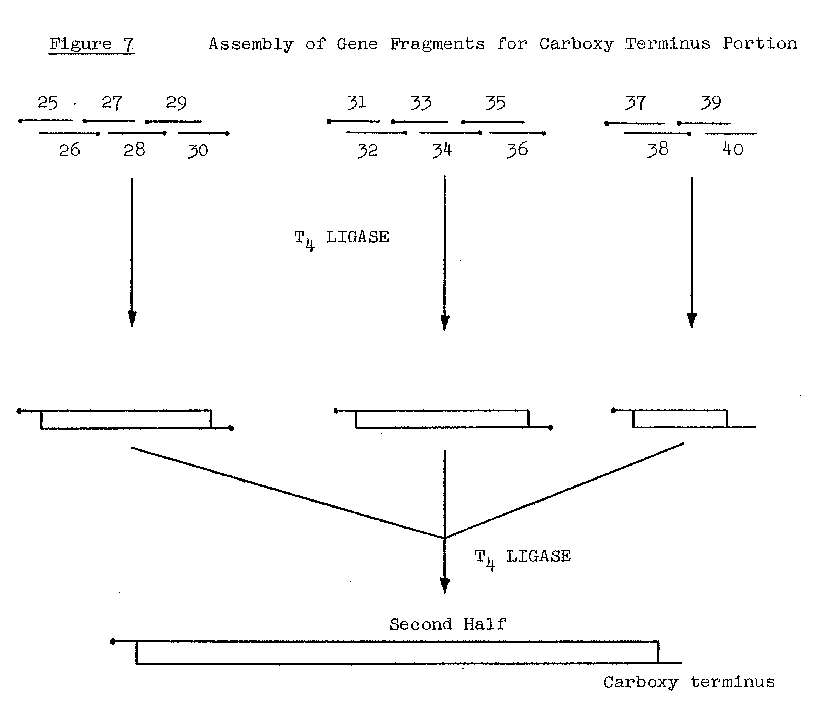

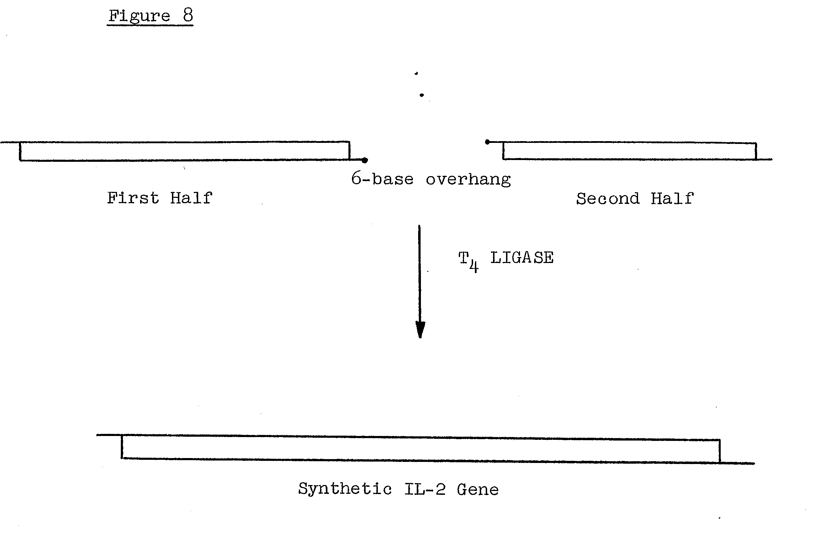

Synthetic DNA coding for mature human interleukin-2 may be prepared by choosing codons according to the genetic code. For ease in preparation and purification, oligodeoxyribonucleotide fragments of, for example, from about 18 to about 24 nucleotides each are prepared separately, and assembled to provide the desired sequences. The first series, when joined in proper sequence, yield the DNA coding strand for expression of mature human interleukin-2 (see Figures 5-7, fragments 1, 3, 5, 7, 9, 11, 13, 15, 17, 19, 21, 23, 25, 27, 29, 31, 33, 35, 37 and 39). The second series when likewise joined in proper sequence, yield the strand complementary to the coding strand (see Figures 5-7, fragments 2, 4, 6, 8, 10, 12, 14, 16, 18, 20, 22, 24, 26, 28, 30, 32, 24, 36, 38 and 40). The fragments of the respective strands preferably overlap such that complementation promotes their self assembly through hydrogen bonding of the chohesive termini of fragment blocks. Following assembly, preferably in a two-step approach (see Figures 6 and 7) the structural gene is completed by ligation in the conventional manner as depicted in Figure 8.

The degeneracy of the genetic code permits substantial freedom in the choice of codons for any given amino acid sequence so that different synthetic structural genes coding for the same compound can be constructed. For present purposes, however, a majority of codons was used known to be preferred in the expression of microbial genomes. Thus the E. coli codon preferences were judiciously combined with preferred codons for yeast expression so that the genes will be suitable for bacterial and yeast expression vectors. The gene sequence selected also provides convenient restriction sites which facilitate its subsequent analysis. Furthermore, the selected sequence for mature human interleukin-2 was computer-scanned to ensure the absence of self-complementary and repeated sequences which might interfer with proper ligation of the oligonucleotides during assembly.

For expression in E. coli, the coding sequence for the interleukin-2 polypeptide is preceded by an ATG initiation codon and followed by a single TGA translational stop codon. EcoRl termini were placed at the beginning and at the end of the gene for convenient insertion into expression vectors. A SalI site was also included following the coding sequence as an alternative site for insertion into the expression vector and to facilitate rapid Maxam-Gilbert sequencing of the gene once it is cloned.

### Preparation of the Expression Vector

A plasmid, phage DNA or other DNA sequence which is able to replicate in the host cell and transform may be referred to herein as an expression vehicle, expression vector, or vector for the purposes of this invention. These vectors are characterized by one or a small number of endonuclease recognition or restriction sites at which DNA sequences may be cut in a determinable fashion without attendent loss of an essential biological function of the DNA, e.g., replication, production of coat proteins or loss of promoter or ribosomal binding sites, and which contain a marker suitable for use in the identification of transformed cells, e.g., ampicillin resistance.

Preferred expression vectors used in the practice of the present invention further comprise hybrid ribosome binding sites. Ribosome binding sites (RBS) from which the hybrid RBS are derived, comprise RNA sequences encoded by the DNA. RBSs are necessary for the initiation of translation in a host cell and consist essentially of (1) an ATG translation initiation codon for the amino acid methionine (all known E. coli gene products begin with the amino acid methionine which may or may not be subsequently cleaved off); (2) a sequence of 3 to 9 bases which are complementary to bases at the 3' end of 16S ribosomal RNA known as Shine-Delgarno (SD) sequence [see Nature 254, 34 (1975)]; and (3) a sequence of bases between these two known as linker region.

The expression vectors forming the preferred embodiment of this invention are derivatives of the plasmid pBR 322 containing the $P_L$ promoter isolated from bacteriophage lambda DNA and inserted between the $tet^R$ and the $amp^R$ genes. $P_L$ is the promoter of choice since it is a very strong promoter that can be controlled efficiently and conveniently by the lambda cI repressor. The gene encoding the repressor carries a mutation, cIts2 or cIts857, which renders the repressor temperature-sensitive. At 30°C the repressor functions normally, and from about 37°C to about 42°C it is inactivated. Thus, the $P_L$ promoter is repressed (turned-off) at 30°C and derepressed (turned-on) at 42°C. The ability to control the $P_L$ promoter allows one to grow the culture at about 30°C to about 36°C without expression of the gene product and to produce the desired mature human IL-2 by shifting the temperature to about 37-42°C at an optimal moment.

The preferred vector used in the present invention also contains an EcoR1 restriction site distal (downstream in the 3'-direction) to the SD sequence, providing a means of constructing different hybrid RBSs. Once the hybrid RBS is constructed using the EcoR1 site to join the SD sequence to the ATG coding sequence of the IL-2 coding gene, it can be further modified by restricting with EcoR1,

filling-in the termini with Klenow Polymerase I and joining the two resulting ends by blunt-end ligation with $T_4$ DNA ligase.

It is also within the skill of the art to utilize other control elements to produce alternative expression vectors in conjunction with the novel structural gene for mature human IL-2 provided by the present invention. Obviously some modification may be required in the termini provided to allow insertion of the gene into the requisite plasmids. Suitable systems for use in bacteria in accordance with the broadest aspect of this invention include the lac promoter-operator system, the arabinose operon (phi 80 dara) or the colicine El, galactose, alkaline phosphatase or tryptophan operons. Similarly the ADH system can be employed to provide expression in yeast.

## The Microorganism

A large number of unicellular microorganisms are known in the art as being suitable for transformation. Particular microorganisms include bacteria, fungi, and algae. Preferred organisms for transformation include bacteria such as strains of E. coli; bacillaceae, such as Bacillus subtilis and the like. Yeasts form a further preferred group of microorganisms for transformation.

In a preferred embodiment of this invention, the microorganism employed as the recipient in the transformation procedures is E. coli K-12 strain 294 as described in British Patent Publication No. 2055382A. Strains of this microorganism have been deposited with the American Type Culture Collection, ATCC Accession Nos. 31446 and 31448 deposited October 28, 1978. Other suitable E. coli strains may also be employed, such as E. coli MA 210 or RR I.

The present invention is further illustrated by the following examples.

Example 1

Plasmid pRC23 was constructed by ligating synthetic oligonucleotides comprising a "consensus" RBS [Scherer et al., Nucleic Acids Research, $\underline{8}$, 3895 (1980)] to a 250 bp BglII - HaeIII fragment containing the lambda $P_L$ promoter, and inserting the ligation product into plasmid pRC2 as shown in Figure 1. The details of this construction are described below.

In order to isolate the 250 base pair (bp) DNA fragment containing the lambda $P_L$ promoter, 1 μg of a 450 bp BglII - HpaI DNA fragment (from bp No. 35260 to 35710 of the November 1981 version of the lambda DNA sequence) was digested with HaeIII and the products were isolated by preparative gel electrophoresis in 5% polyacrylamide. About 200 ng of the 250 bp BglII - HaeIII fragment was ligated to 60 pmoles each of the synthetic oligonucleotides shown in Figure 1 which comprise most of the "consensus" ribosome-binding site sequences generated by computer analysis as described by Scherer et al. (loc. cit.). The ligated molecules were digested with BglII and EcoRl (to eliminate oligomers) and purified by gel electrophoresis. The ligated products were then ligated into pRC2 which also had been digested with BglII and EcoRl. pRC2 is a derivative of pBR322 with a BglII site located adjacent to the EcoRl site (see Figure 1). Transformation of strain RRl(pRK248cIts) was performed using standard methods and transformants were selected on media containing ampicillin (50 μg/ml) at 30°C. 50 transformants were obtained, DNA was isolated from 8 of those and analyzed by digesting with HincII. 6 of the 8 showed the expected restriction pattern and Maxam-Gilbert nucleotide sequence analysis of one of these confirmed the expected construction (designated pRC23).

As outlined in Figure 6, the oligonucleotides are assembled in two stages. Oligonucleotides 1-24 are ligated following the scheme in Figure 6 to generate fragment IL-2 N, the amino-terminal portion of the IL-2 gene. Likewise, oligonucleotides 24-40 are ligated to generate fragment IL-2 C, the carboxy-terminal portion of the gene as seen in Figure 7.

Specific procedures for the aforesaid ligations are set forth below:

20 pmoles each of the synthetic fragments 1-40 are phosphorylated with 5 units of polynucleotide kinase in 10 µl reaction mixtures containing 50 mM Tris-HCl (pH 7.5), 10 mM $MgCl_2$, 0.1 mM EDTA, 5 mM DTT, 0.1 mM spermidine-HCl, 12.5 m ATP, and 37.5 µC [$\gamma$-32p] ATP (carrier-free) at 37°C for 30 minutes. The reactions are terminated by heating at 65°C for 5 min. followed by the addition of 5 µl of 0.5 M EDTA, 5 µl of 10 mg/ml tRNA, and 190 µl of 5 M ammonium acetate. The nucleic acids are ethanol precipitated, resuspended in 200 µl of 0.3 M sodium acetate, ethanol precipitated again, dried under a vacuum and resuspended in 10µl of 1 mM Tris (pH 7.4), 0.1 mM EDTA (0.1 x TE).

The two portions of the IL-2 gene (IL-2 N and IL-2 C) are assembled separately following the schemes outlined in Figures 6 and 7. 8 pmoles of each of the phosphorylated oligonucleotides are ligated with 4 units of T4 DNA ligase in reaction mixtures containing 50 mM Tris (pH 7.4), 10 mM $MgCl_2$, 10 mM ATP at 15°C for 30 to 60 minutes. At the end of the sequential ligations, an additional 200 units of ligase is added and incubation is continued at 4°C for 2 days. The ligation reactions are terminated by heating at 65°C for 5 minutes, then chilled on ice. The ligated molecules are digested with EcoRl, ethanol precipitated, and separated by electrophoresis on a 8% polyacrylamide gel. The 250 bp product comprising the N-terminal portion of the IL-2 gene, and the 170 bp product comprising the C-terminal portion are identified following autoradiography, recovered from the gel, ethanol precipitated twice, dried,

0119621

and resuspended in 10 μl of 0.1 x TE.

For direct expression of the IL-2 gene product in E. coli, the EcoRl-SalI fragment containing the IL-2 gene is inserted into the expression vector pRC23 which has been digested with EcoRl and SalI. pRC23 is a derivative of pBR322 containing the bacteriophage lambda $P_L$ promoter and a synthetic SD sequence adjacent to the EcoRl site (see Figure 1). Joining the IL-2 sequence at this EcoRl site creates a very favorable ribosome-binding site for efficient translation initiation. Transcription initiation from the strong $P_L$ promoter is conveniently and efficiently controlled by the temperature-sensitive cI repressor encoded by the low-copy number plasmid pRK248 cIts.

Specific procedures for constructing the aforesaid recombinant plasmid are set forth below:

2 μl of the purified IL-2 N and IL-2 C fragments are ligated together in a 10 μl reaction mixture as described above with 200 units of T4 DNA ligase at 15°C for 15 hrs. The reaction is terminated by heating the mixture to 65°C for 5 minutes, then chilled on ice. The ligated molecules are digested with EcoRl and SalI, and then ligated as described above to 200 ng of the vector pRC23 (which has also been digested with EcoRl and SalI). The ligation mixture is used to transform strain RRI (pRK248cIts) following standard procedures. Transformants are selected on media containing ampicillin (50 μg/ml). The transformants containing the IL-2 sequence inserted into the vector as expected are determined by restriction analysis of the isolated plasmid DNA. The entire IL-2 coding sequence cloned in the expression vector is then confirmed by Maxam-Gilbert nucleotide sequence analysis.

To test for expression of the cloned synthetic IL-2 gene, two approaches are taken:

1)    analysis of the cell-free translation products encoded by pRC23/IL-2 and

2)    assay of lysates of induced cells containing pRC23/ IL-2 for IL-2 bioactivity. The details of these experiments are described below.

1)    Purified pRC23/IL-2 DNA (along with control DNA) is added to a coupled in vitro transcription-translation system [Kung et al., Arch. Biochem. Biophys. _195_, 396 (1979)] containing $^{35}$S-methionine and the resulting products are analyzed by SDS-polyacrylamide gel electrophoresis followed by fluorography.

2)    Strain RRI (pRK248cIts, pRC23/IL-2) is grown in M9-glucose media at 30°C to about 2 x $10^8$ cells/ml, then induced at 42°C for 2 hrs. 1 ml samples are taken and the cells are collected by centrifugation. The cells are resuspended in 50 µl of 50 mM Tris (pH 7.4), 10% sucrose and quickly frozen in a dry ice/ethanol bath. The cells are thawed and kept at 0-4°C. 5 µl of lysis mix (1 vol of 5 m NaCl, 1 vol 0.5 M EDTA, 1 vol 1 M spermidine-HCl, and 2 vol 5 mg/ml lysozyme) is added, and after 30 minutes on ice, the mixture is incubated at 37°C for 2 minutes. Lysis is complete after an additional cycle of freezing and thawing. Cell debris is removed by centrifugation and the lysates are quickly frozen in a dry ice/ethanol bath. The lysates are assayed for IL-2 biological activity as described by Gillis et al., J. Immunol. _120_, 2027 (1978).

Purification of mature human IL-2 from the lysate can be accomplished using chromatography procedures well known in the art. Such procedures include affinity chromatography employing IL-2 polyclonal or monoclonal antibodies, column chromatography or preferably reverse phase HPLC-procedures (see A. Stern, loc. cit.). Combination of such chromatography methods may also be employed. In this manner, recombinant mature human IL-2 can be obtained in homogeneous form suitable for inclusion in dosage form for therapeutic use.

The recombinant protein when expressed may have a methionine as the amino terminal amino acid. However, in the presence of enzymes in the host microorganism the methionine may be partially or even completely cleaved from the produced product.

## Example 2

### Standard Method for Preparation of 3'-O-Succinylnucleotides

The 5'-O-dimethoxytrityl and N-protected deoxynucleoside (2.5 mmole) is dissolved in dry pyridine (5 ml) and N,N-dimethylaminopyridine (0.3 g). Succinic anhydride (2.0 mmole, 0.2 g) is added and the solution is stirred at room temperature for 24 hrs. After the reaction is completed, solvent is evaporated under vacuum and the dry gum is coevaporated with toluene (10 ml each time) twice. The residue is dissolved in methylene chloride (30 ml) and the solution extracted with citric acid (ice cold). The organic phase is washed twice with water (15 ml each) and then dried over anhydrous sodium sulfate. In order to avoid any detritylation of the product, about 0.3 ml of pyridine is added to the methylene chloride solution. The methylene chloride solution is concentrated to about 10 ml and the succinylated nucleoside is isolated by precipitation into hexane/ether (1:1, v/v; 250 ml). The precipitate is collected by filtration and dried in vacuo.

## Example 3

### Standard Method for Preparation of p-Nitrophenyl Esters of Succinylated Nucleosides

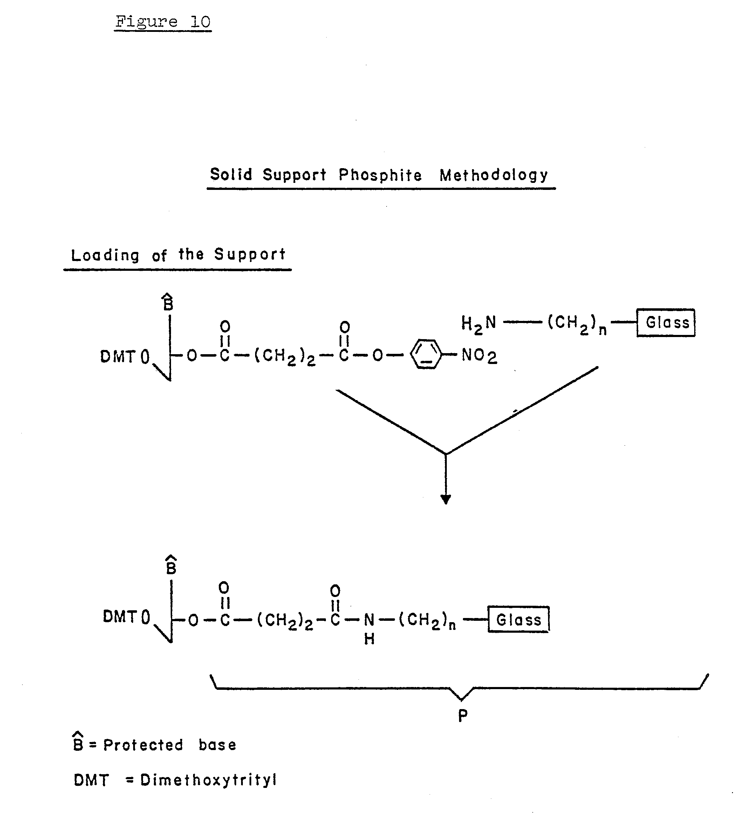

As seen in Figure 10 succinylated nucleoside (1 mmole) is dissolved in dry dioxane (3 ml) containing pyridine (0.3 ml). To the above solution is added dicyclohexylcarbodiimide (DCC, ∿5 mmole, 900 mg) and p-nitrophenol (0.22 g, 1 mmole). The solution is stirred for 2 hrs. The precipitate of dicyclohexylurea is removed by centrifugation. The supernatant is directly used for coupling to the resin as described in the next Example.

## Example 4

### Condensation of Succinylated Nucleosides to the Resin for Solid Support Phosphite Methodology

A 25 ml ($\sim$10 g) sample of Pierce CPG/Long Chain alkylamine controlled pore glass support (pore diameter 500 Å), particle size 125- 177 μ) is suspended in 25 ml dry dimethylformamide (DMF). The solution containing the p-nitrophenyl ester of the succinylated nucleoside is added to the above suspension. The mixture is shaken for 24 hrs. The derivatized resin is isolated, washed with dry DMF (3 times, 30 ml each), dioxane (5 times, 30 ml each), methanol (3 times, 30 ml each) and finally with anhydrous ether (3 times, 30 ml each). Any unreacted amino functions are blocked by reacting the support with a solution of dry pyridine (25 ml), N,N-dimethylamino pyridine (250 mg) and acetic anhydride (5 ml) for 2 hrs. at room temperature. The solid support is filtered and washed successively with methanol (5 times, 30 ml) and anhydrous ether (2 times, 30 ml).

### Determination of Nucleoside Loading on the Support

Approximately a 1 mg sample of the nucleoside-loaded resin is treated with 0.1M toluenesulfonic acid in acetonitrile (1 ml). The trityl carbonium ion released from the resin as a red-orange color is collected and diluted appropriately with acetonitrile. Then the optical density is measured at 498 μm. An average of 4 such measurements provided the extent of loading on the support. The range of loading obtained from several different resin loading experiments was 22-25 μmole of nucleoside/g of the solid support.

0119621

## Example 5

Standard Preparation of 5'-O-Dimethoxytrityl-3'-O-phosphoroamidite Derivatives of Nucleosides

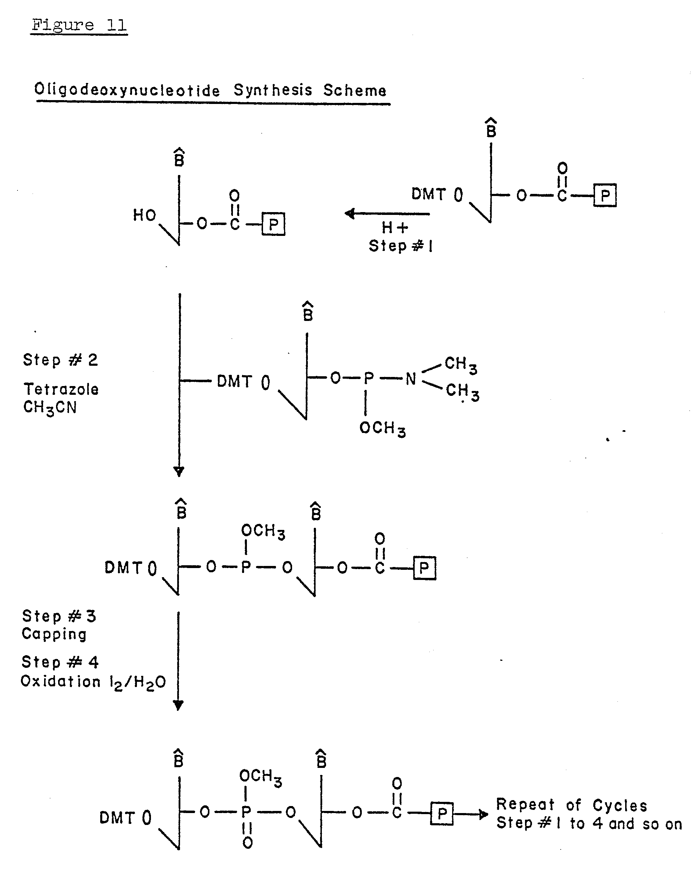

As seen from Figure 11 and following the procedure of Beaucage and Caruthers, Tetrahedron Letters 22, 1859 (1981), a sample of 5'-O-dimethoxytrityl nucleoside (5 mmole) is dissolved in dry and acid-free chloroform. To the solution is added diisopropylethyl-amine (2.74 ml, 15.74 mmole) followed by a gradual addition of dimethylamino-methyl phosphonomonochloridite [$CH_3OP(Cl)N(CH_3)_2$] over 1-2 minutes. The reaction mixture is transferred to a separatory funnel with 175 ml of ethyl acetate. The organic mixture is extracted with saturated brine (4 times, 75 ml). The ethyl acetate phase is dried over anhydrous sodium sulfate. The solution is concentrated to an oil and then redissolved in dry ethyl acetate to obtain 50 ml of the solution. This solution is added dropwise to precooled (ice-acetone temperature) n-hexane (250 ml) for precipitation of the product which is then filtered and dried under high vacuum for 17 hrs. at room temperature. For all four nucleoside derivatives yields of about 80% have been achieved. The samples of the dimethylamino phosphoroamidites were stored under argon at -14°C.

## Example 6

Solid Support Synthesis of Fragment No. 1
5'-AATTCTATGGCTCCGACTT-3' by Phosphite Methodology

Step 1: A sample of 5'-O-dimethoxytritylthymidine-loaded glass support (435 mg, 20 µmole of dimethoxytrityl thymidine/g of support) is detritylated with 0.2 M dichloroacetic acid in dichloromethane (2 ml) by shaking the suspension for 1.5 minutes. The support is washed with dichloromethane (2 times, 5 ml). Dichloroacetic acid treatment is repeated for a few seconds to insure completion of detritylation.

The Support is washed successively as follows:

a) with 1% triethylamine in dichloromethane (2 times, 5 ml)

b) with anhydrous acetonitrile (9 times, 3 ml).

Step 2: A solution of dimethylaminophosphoramidite derivative of dimethoxytrityl thymidine (100 μmole in 2 ml anhydrous acetonitrile) is added to the support followed by the addition of tetrazole solution (250 μmole in 2 ml of acetonitrile). The suspension is shaken for 2 minutes, vacuum filtered and washed with anhydrous acetonitrile (4 x 5 ml).

Step 3: A 2 ml solution of acetic anhydride (0.4 ml) in pyridine containing about 10 mg of N,N-dimethylaminopyridine is shaken with the support for 2 minutes to block any unreacted hydroxyl functions and then the blocked support is washed with acetonitrile (4 times, 5 ml).

Step 4: A solution of iodine (0.2 M) in tetrahydro-furan/water/2,6-lutidine (2:1:1, v/v) is shaken with the blocked support for 0.5 minute, washed with dry acetonitrile (6 times, 5 ml) and washed with dichloromethane (2 times, 5 ml).

Step 1 is then repeated with the product of Step 4. Step 2 is then repeated with the phosphoroamidite deriva-tive of 5'-O-dimethoxytrityl deoxycytidine. Steps 3 and 4 are then repeated.

The cycle of Steps 1 through 4 are then repeated over and over with the addition of each successive nucleoside derivative, thus extending the chain in the 5'-direction by adding corresponding phosphoramide derivatives of deoxy-adenosine (dA), deoxyguanosine (dG), deoxycytidine (dC), dC, thymidine (T), dC, dG, dG, T, dA, T, dC, T, T, dA and dA respectively to yield the desired fragment.

0119621

## Example 7

Deprotection and Release of the Oligonucleotides from the Support

A sample of the oligonucleotide-bound support obtained in Example 6 (150 mg) is treated with a mixture of 1,4-dioxane (0.25 ml), 0.25ml anhydrous triethylamine and 0.125 ml thiophenol for 30 minutes at room temperature. The resin is filtered, washed with methanol (4 x 2 ml) and hydrolyzed with concentrated ammonium hydroxide for 17 hours at 50°C. The resin is pelleted and the supernatant concentrated under vacuum and redissolved in water (1 ml). The solution of the oligonucleotides is passed through a 0.45 micron filter and then fractionated on an HPLC system using a C 18 reverse phase Microbondapak (Waters Assoc.) column under a gradient of acetonitrile 10-30% in 0.05 M triethylammonium acetate, pH 7.0 at 50°C. The well separated dimethoxytrityl containing product is collected, concentrated and treated with 80% aqueous acetic acid for 0.5 hr. at room temperature. After evaporation of acetic acid, the sample is dissolved in water and extracted with ether to remove trityl alcohol. The aqueous layer containing the desired oligonucleotide is separated and examined for its purity on HPLC. A second purification at this stage may be necessary to obtain a homogeneous product. Final criterium of purity is analysis of the $5'-^{32}P$-labeled oligonucleotide on acrylamide gel electrophoresis under denaturing conditions. The nucleotide sequence of the fragments can be ascertained by the Maxam-Gilbert method, Proc. Natl. Acad. Sci. USA $\underline{74}$, 560 (1977).

Following the same approach as described above, synthesis of fragments 13, 14, 15, 16, 17, 18, 19, 20, 21, 22, 23 and 24 of the IL-2 gene is accomplished.

Example 8

Standard Method for Phosphotriester Solid-Phase Synthesis
of Nonadecanucleotide d(CAAGTTCTACCAAGAAGAC), Fragment 3
of IL-2 Gene


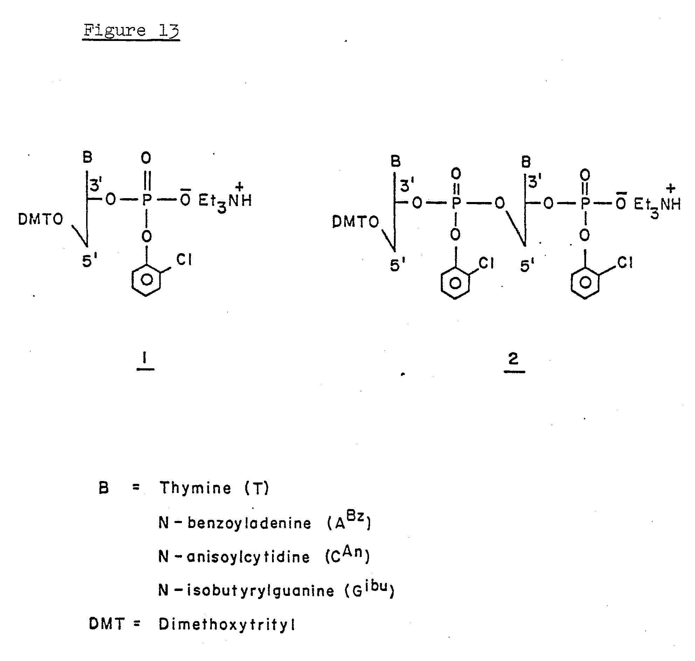

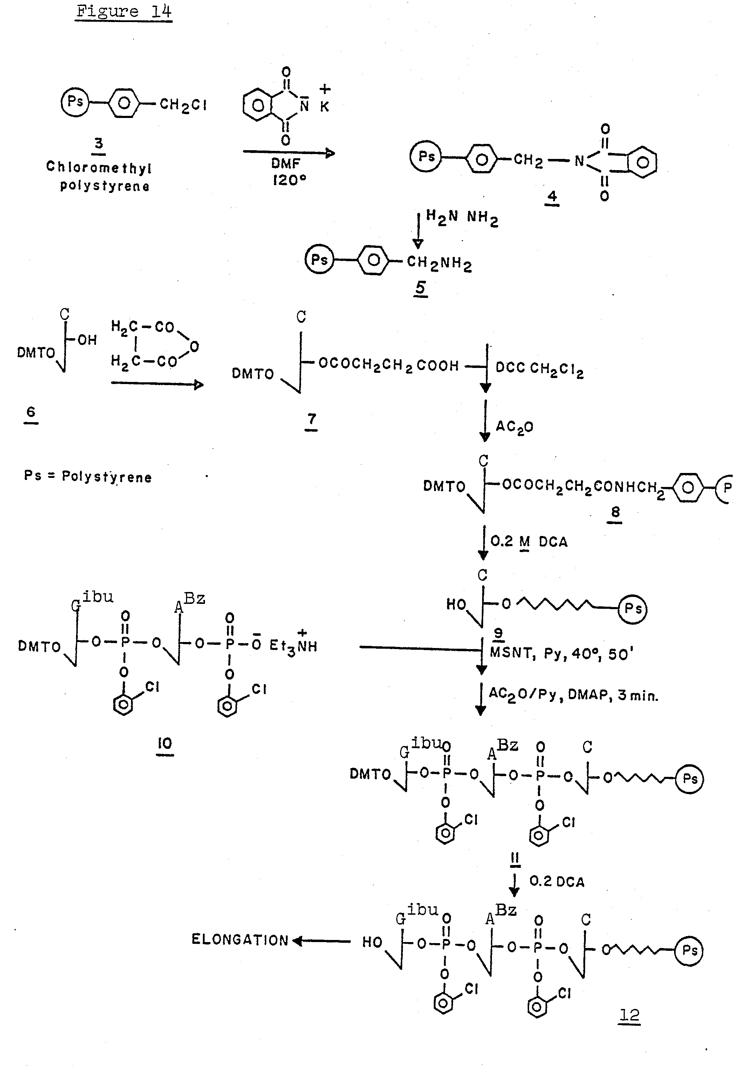

All numbers of compounds referred to in this Example
relate to Figures 13 and 14. The method involves the following steps:

a)   Synthesis of suitably blocked mono- and dinucleotides
     of the general structure (1) and (2) respectively.
b)   Preparation of aminomethyl polystyrene resin (5).
c)   Loading of succinate (7) on to the aminomethyl polystyrene resin (5) and masking of any unreacted amino
     group with acetic anhydride-pyridine.
d)   Removal of 5'-dimethoxytrityl group from polymer
     supported nucleoside (8) with 0.2 M dichloroacetic
     acid (DNA) in $CH_2Cl_2$ to afford resin (9) with a 5'-
     hydroxy function for the coupling reaction.
e)   Condensation of the triethylammonium salt of the
     dimer (10) with (9) to provide polymer supported tri-
     nucleotide (11). Any unreacted 5'-OH groups from (9)
     are masked with 10% acetic anhydride in pyridine.
f)   Detritylation of (11) with 0.2 M DCA to generate a
     new 5'-OH group for the next coupling.


The aforesaid suitably protected mono-/dinucleotides
are synthesized according to procedures known in the art
with slight modifications. 3'-succinate (7) of 5'-dimethoxytrityl-deoxycytidine (6) is prepared according to the method
published by Miyoshi et al., Nucleic Acids Research 8,
5491 (1980).


Aminomethyl Polystyrene Resin (5):

A slurry of commercially available chloromethyl polystyrene (1% cross linked, 0.32 mmol/g Cl, 30 g, 9 mmol) and
potassium phthalimide (2.77 g, 15 mmol) in DMF (250 ml) is heated at 120°C for 20 hours. The resin is then filtered while hot and washed consecutively with hot DMF (200 ml), water (4 x 50 ml), dioxane (3 x 80 ml), ethanol (3 x 80 ml) and ether (3 x 50 ml) to give the phthalimide derivative (4) which is suspended in ethanol (350 ml) and refluxed with hydrazine (5 ml) for 5 hrs. The reaction mixture is filtered and washed with hot ethanol (4 x 70 ml), hot DMF (2 x 70 ml), water (3 x 70 ml), ethanol (3 x 70 ml) and ether (3 x 80 ml). The resin is then dried (28 g) to give aminomethyl polystyrene (5). Picric acid titration showed an amino group concentration of 0.21 mmol/g.

Loading of 5'-Dimethoxy-3'-O-succinyl-deoxycytidine (7) on to the Aminomethyl Resin (5):

To a slurry of 5 g of aminomethyl resin (5) in dry $CH_2Cl_2$ (30 ml) is added monosuccinate (7) (0.97 g, 1.5 mmol), DCC (0.927 g) and dimethylaminopyridine (DMAP) (50 mg) and the reaction mixture is allowed to stir at room temperature for 4 hrs. After filtering, the resin (8) is washed with $CH_2Cl_2$ (4 x 15 ml), MeOH (4 x 15 ml), $CH_2Cl_2$ (4 x 15 ml) and ether (3 x 10 ml). After drying the resin under vacuum (0.1 mm Hg), the concentration of the loaded nucleoside is determined by measuring the absorption of the liberated DMTOH from the support in a 1% BSA solution in $CHCl_3$ [$\lambda_{max.}$ 498, $\varepsilon$ 92100].

Masking of the unreacted amino groups in resin (5), present along with resin (8), is achieved by reacting the mixture with 10% acetic anhydride-pyridine (30 ml) containing DMAP (30 mg) for 30 minutes at room temperature. The resin is then washed with $CH_2Cl_2$, MeOH, $CH_2Cl_2$ and ether. The concentration of the loaded nucleoside may again be determined as described before.

Detritylation of the Resin (8) with 0.2 M Dichloroacetic Acid in $CH_2Cl_2$:

Table 1 below shows the conditions which may be used for the detritylation of each of the dimethoxytritylated nucleoside. A typical experiment can be effected as follows: The resin is swollen in dry $CH_2Cl_2$ (2 ml) for about 30 seconds in a small column (1.3 x 10 cm) and then shaken with 10 ml of 0.2 M DCA for 30-40 seconds followed by a very quick filtration and very quick wash with $CH_2Cl_2$ (3-4 ml). This process is repeated until the washings do not show any color due to DMTOH (see Table 1). The washings are collected and the absorption is measured to determine the concentration of the DMTOH which reflects the concentraton of the loaded mono- or dinucleotide.

After complete detritylation has been achieved, the resin (9) is washed with 0.5 M $Et_3N$ solution in $CH_2Cl_2$, with $CH_2Cl_2$ (3 x 10 ml) and with pyridine (3 x 5 ml).

Table 1

| (DMT)N | Treatment with 0.2 M DCA | Duration of treatment | Washings with $CH_2Cl_2$ |
|--------|--------------------------|-----------------------|---------------------------|
| T | 5 x 10 ml | 20 sec. | 1 x 3 ml |
| C | 5 x 10 ml | 20 sec. | 1 x 3 ml |
| G | 3 x 10 ml | 15 sec. | 1 x 3 ml |
| A | 4 x 10 ml | 15 sec. | 1 x 3 ml |

Coupling of GA Dimer (10) with 5'-Hydroxy-polystyrene Support (9) to give the Polymer-supported GAC Trimer (11):

The 5'-hydroxy resin (9) (57 mg, 0.01 mmol) which has been washed with pyridine is dried under high vacuum (0.005 mm Hg) and by blowing hot air on the column from a heat gun for 2 minutes. The vacuum is carefully released by letting in dry air or argon. To the resin is then added a pyridine solution (0.7 ml) of triethylammonium salt of the GA dimer (10) (0.06 mmol) followed by the addition of mesitylenesulfonyl-3-nitro-triazole (MSNT) (0.18 mmol).
The column is properly stoppered and heated at 40°C for
50 min. The reaction mixture is filtered and washed with
pyridine (3 x 3 ml). Unreacted 5'-OH groups are masked by
reacting the resin with 10% $Ac_2O$/Py containing DMAP (10 mg)
for 3 minutes at room temperature. The resin is filtered
and successively washed with pyridine (2 x 2 ml), $CH_2Cl_2$
(2 x 5 ml), MeOH (2 x 5 ml) and again with $CH_2Cl_2$ (2 x 5 ml)
to afford the polystyrene polymer supported GAC trimer (11).

The 5'-dimethoxytrityl group of the resin-bound trimer
(11) is removed with 0.2 M DCA as described above (Table 1)
and 5'-unprotected resin-bound trimer (12) resulting therefrom is extended to the desired nonadecamer by repeating
each cycle with a dimer or a monomer of choice.

Deblocking and Purification:

After the last coupling reaction, the resin (50 mg)
is treated with a 0.5 molar solution of $N^1,N^1,N^3,N^3$-tetra-
methylguanidinium pyridine-2-carboxaldoximate (1 ml) in 80%
dioxane-$H_2O$ for 15 hours at room temperature according to
the procedure of Reese et al., Tet. Letters 19, 2727 (1978).
The solvent is then evaporated, the residue left is treated
with concentrated ammonia (28%, 4 ml) at 60°C for 8 hrs.
After centrifugation, the supernatant is concentrated to a
volume of ca 0.5 ml which is dissolved in 0.05 M TEAB
(1.5 ml) and passed through a Sephadex G-50 column using
0.05 M TEAB as the mobile phase. Fractions of 6 ml are
collected and the absorption is measured on a Beckman UV
spectrophotometer Model 34. Fractions 30-33 containing the
product were collected to give 100.8 ODs. 50 ODs of this
product was purified by HPLC on a microbondapak $C_{18}$ column
using a linear gradient of 7.5 to 37.5% acetonitrile (pH
7.8) over a period of 12 minutes. Peak 2 eluting with 31%
$CH_3CN$ contained the dimethoxytrityl product and was collected to give 30.2 ODs. The solvent was evaporated, and the
residue was detritylated with 80% acetic acid (20 minutes
at room temperature). The acetic acid was evaporated and the residue coevaporated with toluene (3 x 1 ml) to remove residual acetic acid. The resulting residue was then dissolved in water (2 ml) and extracted with ether (3 x 3 ml) to remove any organic impurities. The aqueous solution was carefully concentrated to a volume of 400 µl, filtered and purified on a microbondapak $C_{18}$ HPLC column using a linear gradient of 7.5-37.5% acetonitrile. The peak eluting with 20.6% acetonitrile was collected and further purified by electrophoresis on an acrylamide gel in the presence of 7 M urea. The slowest moving band was eluted with buffer and after labelling the 5'-hydroxyl group with $\gamma[^{32}P]$-ATP the sequence of the nonadecanucleotide fragment obtained was confirmed by Maxam-Gilbert sequence analysis.

Following the same procedure as described above fragments 2, 4, 5, 6, 7, 8, 9, 10, 11, 12, 25, 26, 27, 28, 29, 30, 31, 32, 33, 34, 35, 36, 37, 38, 39 and 40 of the IL-2 gene were synthesized.

CLAIMS:

1. A double-stranded polydeoxyribonucleotide coding for mature human IL-2 and having cohesive ends each one representing a part of an endonuclease recognition site.

2. A polydeoxyribonucleotide according to claim 1 wherein the ends represent parts of recognition sites of different endonucleases.

3. A polydeoxyribonucleotide according to claim 2 wherein the end coding for the amino terminus of said mature human IL-2 belongs to an EcoRl recognition site and the end coding for the carboxy terminus of IL-2 belongs to a SalI recognition site.

4. A polydeoxyribonucleotide according to claim 1 of the DNA sequence given in Figure 2 hereinbefore.

5. A DNA sequence coding for mature human IL-2 operably linked with a DNA sequence capable of effecting microbial expression of said mature human IL-2.

6. A structural gene coding for mature human IL-2 of the DNA sequence given in Figure 3 hereinbefore.

7. A replicable microbial expression vehicle capable, in a transformant microorganism, of expressing mature human IL-2.

8. The expression vehicle of claim 7 characterized in that it contains a promoter and operator derived from bacteriophage lambda, a hybrid ribosome binding site and a DNA sequence coding for mature human IL-2.

9. The expression vehicle of claim 8 wherein the promoter and operator are $P_L$ and $O_L$.

10. The expression vehicle of claim 9 which is pRC 23/ EcoRl-SalI into which has been inserted a structural gene coding for mature human IL-2.

11. A microorganism transformed with an expression vehicle as claimed in claims 7-10.

12. The microorganism according to claim 11 which is a transformed E. coli strain.

13. Human Met-IL-2.

14. A method for the production of recombinant mature human IL-2 comprising growing a microorganism as claimed in claim 11 or 12, inducing expression of the IL-2 gene, lysing the microorganism upon completion of the induction period and separating said mature human IL-2 from the cellular constituents of said microorganism.

15. The method of claim 14 wherein said microorganism is grown at a temperature in the range of about 30 to 36°C and gene expression is induced by raising the temperature to about 42°C.

16. The method of claim 14 or 15 wherein said microorganism expresses human Met-IL-2.

Figure 1

Figure 2     2/14     0119621

```
                                                                50
AATTCTATG GCTCCGACTT CAAGTTCTAC CAAGAAGACC CAGCTTCAAT
   GATAC CGAGGCTGAA GTTCAAGATG GTTCTTCTGG GTCGAAGTTA


                                                               100
TAGAACATTT ACTTCTAGAT TTACAÁATGA TTCTGAATGG TATCAACAAT
ATCTTGTAAA TGAAGATCTA AATGTTTACT AAGACTTACC ATAGTTGTTA


                                                               150
TATAAGAATC CAAAGCTTAC TCGTATGTTG ACCTTTAAAT TCTATATGCC
ATATTCTTAG GTTTCGAATG AGCATACAAC TGGAAATTTA AGATATACGG


                                                               200
TAAGAAGGCT ACTGAATTAA AACACCTGCA GTGTTTAGAA GAAGAGCTCA
ATTCTTCCGA TGACTTAATT TTGTGGACGT CACAAATCTT CTTCTCGAGT


                                                               250
AACCGTTAGA AGAAGTTCTG AATCTGGCTC AATCTAAAAA CTTCCATTTA
TTGGCAATCT TCTTCAAGAC TTAGACCGAG TTAGATTTTT GAAGGTAAAT


                                                               300
CGTCCACGAG ATCTTATCTC TAATATTAAC GTAATCGTTT TGGAACTTAA
GCAGGTGCTC TAGAATAGAG ATTATAATTG ·CATTAGCAAA ACCTTGAATT


                                                               350
AGGATCCGAA ACTACCTTCA TGTGTGAATA TGCTGACGAA ACCGCTACGA
TCCTAGGCTT TGATGGAAGT ACACACTTAT ACGACTGCTT TGGCGATGCT


                                                               400
TCGTAGAATT TCTTAATCGA TGGATTACTT TCTGTCAATC TATTATCTCT
AGCATCTTAA AGAATTAGCT ACCTAATGAA AGACAGTTAG ATAATAGAGA


ACCTTAACTT GAGTCGACG
TGGAATTGAA CTCAGCTGCT TAA
```

Figure 3

```
                                                                    55
GCT CCG ACT TCA AGT TCT ACC AAG AAG ACC CAG CTT CAA TTA GAA
Ala Pro Thr Ser Ser Ser Thr Lys Lys Thr Gln Leu Gln Leu Glu


                                                                    100
CAT TTA CTT CTA GAT TTA CAA ATG ATT CTG AAT GGT ATC AAC AAT
His Leu Leu Leu Asp Leu Gln Met Ile Leu Asn Gly Ile Asn Asn


                                                                    145
TAT AAG AAT CCA AAG CTT ACT CGT ATG TTG ACC TTT AAA TTC TAT
Tyr Lys Asn Pro Lys Leu Thr Arg Met Leu Thr Phe Lys Phe Tyr


                                                                    190
ATG CCT AAG AAG GCT ACT GAA TTA AAA CAC CTG CAG TGT TTA GAA
Met Pro Lys Lys Ala Thr Glu Leu Lys His Leu Gln Cys Leu Glu


                                                                    235
GAA GAG CTC AAA CCG TTA GAA GAA GTT CTG AAT CTG GCT CAA TCT
Glu Glu Leu Lys Pro Leu Glu Glu Val Leu Asn Leu Ala Gln Ser


                                                                    280
AAA AAC TTC CAT TTA CGT CCA CGA GAT CTT ATC TCT AAT ATT AAC
Lys Asn Phe His Leu Arg Pro Arg Asp Leu Ile Ser Asn Ile Asn


                                                                    325
GTA ATC GTT TTG GAA CTT AAA GGA TCC GAA ACT ACC TTC ATG TGT
Val Ile Val Leu Glu Leu Lys Gly Ser Glu Thr Thr Phe Met Cys


                                                                    370
GAA TAT GCT GAC GAA ACC GCT ACG ATC GTA GAA TTT CTT AAT CGA
Glu Tyr Ala Asp Glu Thr Ala Thr Ile Val Glu Phe Leu Asn Arg


                                                                    415
TGG ATT ACT TTC TGT CAA TCT ATT ATC TCT ACC TTA ACT
Trp Ile Thr Phe Cys Gln Ser Ile Ile Ser Thr Leu Thr
```

Figure 4

Restriction Map of Synthetic IL-2 Gene *

EcoRl   AluI   Xba   HindIII   DdeI   PstI   SacI   HinfI   BglII   BamHI   PvuI   ClaI   SalI   EcoRl

* Gene designed using codons believed preferable for expression in E. coli

Figure 5                                    0119621

Fragments for the Synthesis of IL-2 Gene

```
         5'                      3'
   1.    AATTCTATGGCTCCGACTT           19-mer
   2.    AACTTGAAGTCGGAGCCATAG         21-mer
   3.    CAAGTTCTACCAAGAAGAC           19-mer
   4.    AGCTGGGTCTTCTTGGTAG           19-mer
   5.    CCAGCTTCAATTAGAACATTTA        22-mer
   6.    TAGAAGTAAATGTTCTAATTGA        22-mer

   7.    CTTCTAGATTTACAAATGATTCT       23-mer
   8.    CCATTCAGAATCATTTGTAAATC       23-mer
   9.    GAATGGTATCAACAATTATAA         21-mer
  10.    GGATTCTTATAATTGTTGATA         21-mer
  11.    GAATCCAAAGCTTACTCGTAT         21-mer
  12.    GTCAACATACGAGTAAGCTTT         21-mer

  13.    GTTGACCTTTAAATTCTATATG        22-mer
  14.    CTTAGGCATATAGAATTTAAAG        22-mer
  15.    CCTAAGAAGGCTACTGAATTAA        22-mer
  16.    GGTGTTTTAATTCAGTAGCCTT   .    22-mer
  17.    AACACCTGCAGTGTTTAGAA          20-mer
  18.'   CTCTTCTTCTAAACACTGCA          20-mer

  19.    GAAGAGCTCAAACCGTTAGAA         21-mer
  20.    AACTTCTTCTAACGGTTTGAG         21-mer
  21.    GAAGTTCTGAATCTGGCT            18-mer
  22.    AGATTGAGCCAGATTCAG            18-mer
  23.    CAATCTAAAAACTTCCATTT          20-mer
  24.    GGACGTAAATGGAAGTTTTT          20-mer

  25.    ACGTCCACGAGATCTTAT            18-mer
  26.    TTAGAGATAAGATCTCGT            18-mer
  27.    CTCTAATATTAACGTAATCGTTT       23-mer
  28.    GTTCCAAAACGATTACGTTAATA       23-mer
  29.    TGGAACTTAAAGGATCCGAAA         21-mer
  30.    AGGTAGTTTCGGATCCTTTAA         21-mer

  31.    CTACCTTCATGTGTGAATATG         21-mer
  32.    CGTCAGCATATTCACACATGA         21-mer
  33.    CTGACGAAACCGCTACGAT           19-mer
  34.    TCTACGATCGTAGCGGTTT           19-mer
  35.    CGTAGAATTTCTTAATCGATG         21-mer
  36.    GTAATCCATCGATTAAGAAAT         21-mer

  37.    GATTACTTTCTGTCAATCTATTAT      24-mer
  38.    GTAGAGATAATAGATTGACAGAAA      24-mer
  39.    CTCTACCTTAACTTGAGTCGACG       23-mer
  40.    AATTCGTCGACTCAAGTTAAG         21-mer
```

Figure 6    Assembly of Gene Fragments for Amino Terminus Portion

Figure 7    Assembly of Gene Fragments for Carboxy Terminus Portion

Second Half

Carboxy terminus

## Figure 8

First Half    6-base overhang    Second Half

$T_4$ LIGASE

Synthetic IL-2 Gene

Figure 9

PL → $\underset{\text{AGGAGGAATTCTATG}}{\overset{\text{SD}}{\phantom{x}}}$ - IL-2

Figure 10

## Solid Support Phosphite Methodology

### Loading of the Support

$\hat{B}$ = Protected base

DMT = Dimethoxytrityl

Figure 11

Oligodeoxynucleotide Synthesis Scheme

Figure 12

Deprotection and Release of Oligonucleotides from the Support

Figure 13

1

2

B  =  Thymine (T)

N – benzoyladenine (A$^{Bz}$)

N – anisoylcytidine (C$^{An}$)

N – isobutyrylguanine (G$^{ibu}$)

DMT =  Dimethoxytrityl

0119621

Figure 14

Ps = Polystyrene

0119621

European Patent
Office

**EUROPEAN SEARCH REPORT**

Application number

| | | | |
|---|---|---|---|
| **DOCUMENTS CONSIDERED TO BE RELEVANT** | | | EP 84103000.0 |

| Category | Citation of document with indication, where appropriate, of relevant passages | Relevant to claim | CLASSIFICATION OF THE APPLICATION (Int. Cl. ³) |
|---|---|---|---|
| X,P | EP - A1 0 091 539 (AJINOMOTO CO., INC., JAPANESE FOUNDATION FOR CANCER RESEARCH) <br><br> * Claims 18,19,21,29; fig. 2(a)* <br><br> -- | 1,5-7, 12-14, 16 | C 12 N 15/00 <br><br> C 12 P 19/34 <br><br> C 12 P 21/00// <br><br> C 12 R 1/19 |
| A,D, P | NATURE, vol. 302, March 24, 1983 (New York, USA) <br><br> T. TANIGUCHI: "Structure and expression of a cloned cDNA for human interleukin-2" pages 305-310 <br><br> * Totality * <br><br> ---- | 1,5-8, 12-14, 16 | |
| | | | **TECHNICAL FIELDS SEARCHED (Int. Cl. ³)** <br><br> C 12 N <br><br> C 12 P |

The present search report has been drawn up for all claims

| Place of search | Date of completion of the search | Examiner |
|---|---|---|
| VIENNA | 12-06-1984 | WOLF |

EPO Form 1503. 03.82

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document